# EUROPEAN PATENT APPLICATION

(11) **EP 4 231 224 A1**
(43) Date of publication of application: **23.08.2023**
(21) Application number: 21880036.5
(22) Date of filing: 11.10.2021
(51) Int. Cl.: G06Q 50/22, G16H 40/00

(54) **ASSISTANCE SYSTEM FOR PERSON REQUIRING NURSING CARE**

(30) Priority: 14.10.2020 JP 2020172886
(71) Applicant: Aikomi Co., Ltd., Yokohama-shi, Kanagawa 220-0012 (JP); Sumitomo Pharma Co., Ltd., Osaka 541-0045 (JP)
(72) Inventor: Kato Junichi, Yokohama-shi, Kanagawa 220-0012 (JP); Hird Nicholas William, Yokohama-shi, Kanagawa 220-0012 (JP); Hori Seiji, Tokyo 103-6012 (JP)
(74) Representative: Zimmermann & Partner Patentanwälte mbB
(86) International application number: PCT/JP2021/037530
(87) International publication number: WO 2022/080297

(57) **Abstract**

There is provided a care-needing person assistance system that can efficiently collect stimulus information. The care-needing person assistance system includes a stimulus information storage means that stores stimulus information about a stimulus for at least one of five senses of a care-needing person, and a value exchange means that provides compensation for provision of stimulus information to a provider who provides new stimulus information to be additionally stored in the stimulus information storage means.

## Description

### Technical Field

The present invention relates to a care-needing person assistance system.

### Background Art

With the advent of an aging society, the number of care-needing persons needing nursing care and assistance by care assistants has increased, whereas the shortage of care assistants is being perpetuated due to physical and mental burdens of care or assistance. Considering an approach to the care of a care-needing elderly person in the future is a common challenge around the world including our country with a high rate of elderly persons.

Behavioral and psychological symptoms include behavioral disorders such as wandering, and verbal abuse or violence, and psychological symptoms such as anxiety, sleeplessness, and hallucination or delusion, and before or after such a disorder or symptom appears, non-drug therapy including exercise therapy and psychotherapy to be performed without drug administration is primarily selected.

In order to improve the behavioral and psychological symptoms using such non-drug therapy, it is necessary to carry out good communication between a care-needing person and a care assistant or a family of the care-needing person, and stimulate various senses including a visual sense, an auditory sense or a tactile sense according to individual condition of the care-needing person.

In Patent Literature 1, there is proposed a technique of providing stimulus information for stimulating at least one of five senses of a care-needing person without resorting to drug therapy to thereby properly improve behavioral and psychological symptoms of the care-needing person.

### Citation List

### Patent Literature

Patent Literature 1: Japanese Patent Application No. 2019-75959

### Summary of Invention

### Technical Problem

In order to effectively provide the stimulus information to the care-needing person using techniques of this type, it is necessary to efficiently collect the stimulus information which makes it possible to properly improve the behavioral and psychological symptoms.

However, since the collection of the stimulus information is performed mainly on the basis of kindness of an assistant of a care-needing person and a family of the care-needing person, a range of collecting the stimulus information tends to be limited, which may make it impossible to efficiently collect the stimulus information.

The present invention has been made in view of the above-described circumstance, and the objective thereof is to provide a care-needing person assistance system that can efficiently collect stimulus information to provide the stimulus information for improving behavioral and psychological symptoms.

### Solution to Problem

In order to achieve the above-described objective, a care-needing person assistance system according to the present invention includes a stimulus information storage means that stores stimulus information about a stimulus for at least one of five senses of a care-needing person, and a value exchange means that provides compensation for provision of stimulus information to a provider who provides new stimulus information to be additionally stored in the stimulus information storage means.

Here, the stimulus information may be at least one of a video, an image, music, a sound and an aroma.

Furthermore, the care-needing person assistance system further includes a stimulus information provision means that provides, to the care-needing person, the stimulus information stored in the stimulus information storage means, and a response detection means that detects a response of the care-needing person to the stimulus by the stimulus information provided by the stimulus information provision means.

In the care-needing person assistance system, the compensation for provision of stimulus information to be provided to the provider may be determined on a basis of a specific response of the care-needing person detected by the response detection means, or the compensation for provision of stimulus information to be provided to the provider may be determined on a basis of a number of times that the new stimulus information is provided to the care-needing person via the stimulus information provision means.

Here, the compensation for provision of stimulus information may be money and/or information regarding a status of provision of the new stimulus information to the care-needing person via the stimulus information provision means.

Furthermore, the value exchange means of the care-needing person assistance system requests use compensation from a user on a basis of access of the user to the stimulus information storage means, and the use compensation is money.

The care-needing person assistance system executes a first step of providing, from among pieces of the stimulus information stored in the stimulus information storage means, first stimulus information enabling mental tension of the care-needing person to be released, a second step of providing, from among the pieces of the stimulus information, any pieces of second stimulus information of pieces of second stimulus information stored in the stimulus information storage means on a basis of an attribute of the care-needing person and detecting an attribute of a piece of the second stimulus information to which the care-needing person responds, from among the any pieces of the second stimulus information provided, and a third step of providing, to the care-needing person, another piece of the second stimulus information having the same attribute as the attribute of the piece of the second stimulus information detected in the second step.

At this time, the care-needing person assistance system may execute a periodic care assistance flow in which, after providing the piece of the second stimulus information detected in the second step to cause the care-needing person to respond to the piece of the second stimulus information and arousing an interest of the care-needing person in the attribute of the piece of the second stimulus information, the other piece of the second stimulus information is provided so that the care-needing person is engaged in the other piece of the second stimulus information.

Here, in the periodic care assistance flow, the first stimulus information is provided to the care-needing person before the second stimulus information is provided to the care-needing person, and third stimulus information enabling the care-needing person to be cooled down is provided after the other piece of the second stimulus information is provided to the care-needing person.

### Advantageous Effect of Invention

According to the present invention, the collection of the stimulus information can be efficiently performed to provide the stimulus information for improving behavioral and psychological symptoms.

### Brief Description of Drawings

[Figure 1] Figure 1 is a block diagram illustrating a schematic configuration of a care-needing person assistance system according to an embodiment of the present invention.
[Figure 2] Figure 2 is a diagram illustrating a schematic configuration of a server of the care-needing person assistance system according to the present embodiment.
[Figure 3] Figure 3 is a diagram illustrating a schematic configuration of a storage of the server of the care-needing person assistance system according to the present embodiment.
[Figure 4] Figure 4 is a block diagram illustrating a schematic configuration of an assistance program to be stored in the storage of the server of the care-needing person assistance system according to the present embodiment.
[Figure 5] Figure 5 is a block diagram illustrating a schematic configuration of a first storage area to be stored in the storage of the server of the care-needing person assistance system according to the present embodiment.
[Figure 6] Figure 6 is a block diagram illustrating a schematic configuration of a first content library of the first storage area to be stored in the storage of the server of the care-needing person assistance system according to the present embodiment.
[Figure 7] Figure 7 is a block diagram illustrating a schematic configuration of a second content library of the first storage area to be stored in the storage of the server of the care-needing person assistance system according to the present embodiment.
[Figure 8] Figure 8 is a block diagram illustrating a schematic configuration of a third content library of the first storage area to be stored in the storage of the server of the care-needing person assistance system according to the present embodiment.
[Figure 9] Figure 9 is a block diagram illustrating a schematic configuration of a second storage area to be stored in the storage of the server of the care-needing person assistance system according to the present embodiment.
[Figure 10] Figure 10 is a diagram illustrating a schematic configuration of a facility device of the care-needing person assistance system according to the present embodiment.
[Figure 11] Figure 11 is a diagram illustrating a schematic configuration of a storage of a facility terminal in the facility device of the care-needing person assistance system according to the present embodiment.
[Figure 12] Figure 12 is a diagram illustrating an overview in the case where a content is displayed on a display by a second step processing module of the facility terminal in the facility device of the care-needing person assistance system according to the present embodiment.
[Figure 13] Figure 13 is a diagram illustrating an overview in the case where a content is displayed on the display by the second step processing module of the facility terminal in the facility device of the care-needing person assistance system according to the present embodiment.
[Figure 14] Figure 14 is a diagram illustrating an overview of a care assistance flow to be executed for the purpose of improving behavioral and psychological symptoms of a care-needing person using the care-needing person assistance system according to the present embodiment.
[Figure 15] Figure 15 is a diagram illustrating an overview of a care assistance preparation flow to be executed using the care-needing person assistance system according to the present embodiment.
[Figure 16] Figure 16 is a diagram illustrating an overview of a periodic care assistance flow to be executed using the care-needing person assistance system according to the present embodiment.

### Description of Embodiment

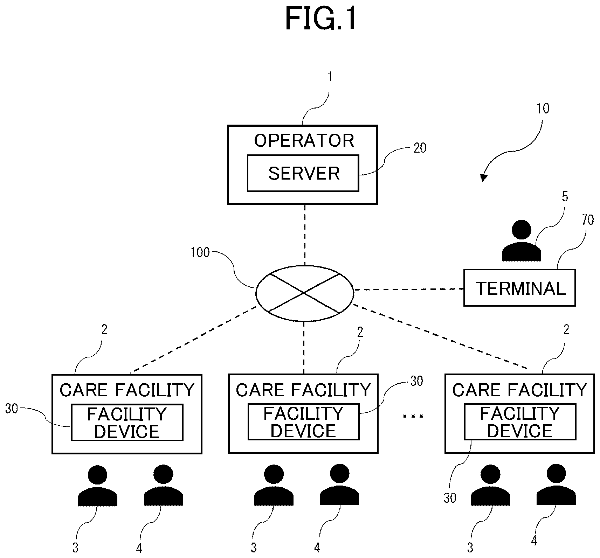

Next, an embodiment of the present invention will be described with reference to Figures 1 to 16.

Figure 1 is a block diagram illustrating a schematic configuration of a care-needing person assistance system according to the present embodiment.

As illustrated in the figure, a care-needing person assistance system 10 includes, as main components, a server 20 deployed in an operator 1, facility devices 30 deployed in respective care facilities 2 which are users, and a terminal 70 deployed in a content provider 5 who is a provider, and the server 20, the facility devices 30 and the terminal 70 are connected to one another via the Internet 100.

In the present embodiment, the care-needing person assistance system 10 is used when a care assistant 3 provides any contents which are pieces of stimulus information, to a care-needing person 4 who developed behavioral and psychological symptoms and is residing in the care facility 2, via the facility device 30, to care for the care-needing person 4.

Here, the care-needing person 4 generally refers to a person aged about 40 years or over who needs care (e.g. nursing care) and recognized as having physical or mental disorder due to particular disease, but in the present embodiment, the care-needing person 4 also includes a person aged 40 years or younger who needs care.

The care assistant 3 generally refers to a person who assists care of the care-needing person 4, and in the present embodiment, the care assistant 3 includes a care assistance expert, but is not limited thereto.

The contents to be provided to the care-needing person 4 stimulate at least one of five senses (a visual sense, an auditory sense, an olfactory sense, a tactile sense, and a gustatory sense) of the care-needing person 4, and in the present embodiment, the contents include a video or an image that stimulates the visual sense, music or a sound that stimulates the auditory sense, an aroma that stimulates the olfactory sense, and the like, and further include stimulating contents used for a cognitive stimulation therapy or a reminiscence therapy.

On the other hand, in the care-needing person assistance system 10, a new content is provided from the content provider 5 to the server 20 of the operator 1 via the terminal 70.

In the present embodiment, the content provider 5 is assumed to be an individual who can create a content that stimulates the care-needing person 4, or a business entity, a local government entity, or a national institution (library or the like) that performs content production.

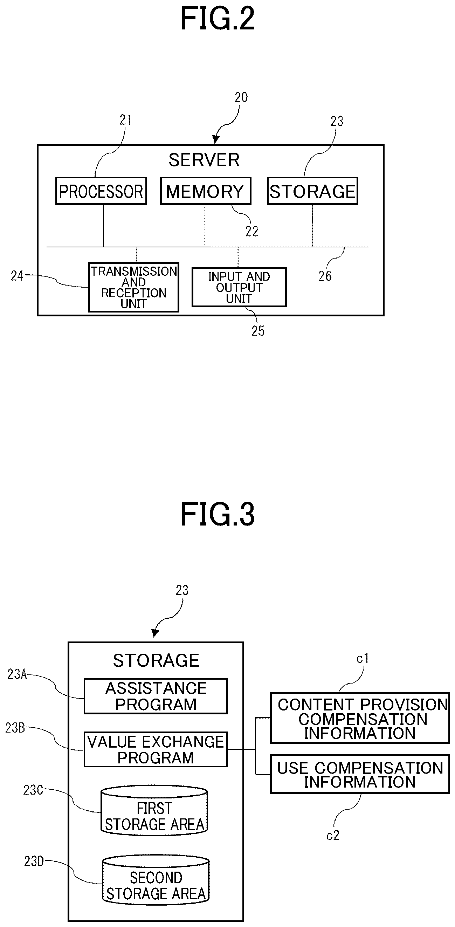

Figure 2 is a diagram illustrating a schematic configuration of the server 20 of the care-needing person assistance system 10 according to the present embodiment. As illustrated in the figure, the server 20 includes, as main components, a processor 21, a memory 22, a storage 23, a transmission and reception unit 24, and an input and output unit 25, and these components are electrically connected to one another via a bus 26.

The processor 21 is a computing device that controls the operation of the server 20 to control exchange of data between elements and perform processing required to execute an application program, for example.

In the present embodiment, the processor 21 is, for example, a central processing unit (CPU), and executes an application program and the like that are stored in the storage 23 and loaded into the memory 22 to perform the processing (which will be described later).

In present embodiment, the memory 22 is implemented by a main storage device constituted by a volatile storage device such as a dynamic random access memory (DRAM).

The memory 22 is used as a work area of the processor 21, and stores a basic input/output system (BIOS) to be executed when the server 20 starts up, various pieces of setting information, and the like.

The storage 23 stores an application program, data used for various types of processing, and the like. In the present embodiment, the storage 23 stores an assistance program that performs various types of processing. The details of the assistance program will be described later.

The transmission and reception unit 24 connects the server 20 to the Internet 100. The transmission and reception unit 24 may include a short-range communication interface such as Bluetooth (registered trademark) and Bluetooth Low Energy (BLE).

In the present embodiment, the server 20 is connected to the facility devices 30 via the transmission and reception unit 24 and the Internet 100.

The input and output unit 25 is connected with an information input device such as a keyboard and a mouse and an output device such as a display, if necessary.

The bus 26 communicates, for example, an address signal, a data signal, and various control signals among the processor 21, the memory 22, the storage 23, the transmission and reception unit 24, and the input and output unit 25 that are connected to the bus 26.

Figure 3 is a diagram illustrating a schematic configuration of the storage 23 of the server 20. As illustrated in the figure, the storage 23 includes an assistance program 23A, a value exchange program 23B which is the value exchange means, a first storage area 23C which is the stimulus information storage means implemented as a storage area provided by the storage 23, and a second storage area 23D similarly implemented as a storage area provided by the storage 23.

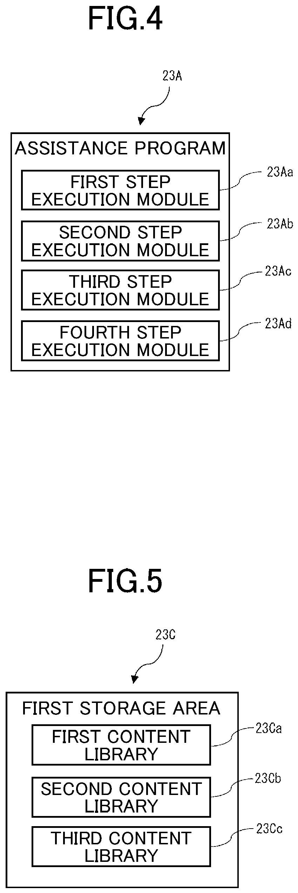

Figure 4 is a block diagram illustrating a schematic configuration of the assistance program 23A. As illustrated in the figure, the assistance program 23A includes a first step execution module 23Aa, a second step execution module 23Ab, a third step execution module 23Ac, and a fourth step execution module 23Ad.

In the present embodiment, the first step execution module 23Aa is a module that extracts any content from a first content library (which will be described later) stored in the first storage area 23C on the basis of a request signal from the facility device 30.

In the present embodiment, the second step execution module 23Ab is a module that extracts any content from a second content library (which will be described later) stored in the first storage area 23C.

In the present embodiment, when the care-needing person 4 shows a specific response at the time when the extracted content is provided to the care-needing person 4 via the facility device 30, and the response is detected by a response detection unit (which will be described later) of the facility device 30, the second step execution module 23Ab detects an attribute (which will be described later) of the content to which the care-needing person 4 shows the response.

In the present embodiment, the third step execution module 23Ac is a module that extracts, from the second content library stored in the first storage area 23C, any other content having the same attribute as the attribute of the content that is detected by the second step execution module 23Ab and to which the care-needing person 4 shows the response.

In the present embodiment, the fourth step execution module 23Ad is a module that extracts any content from a third content library (which will be described later) stored in the first storage area 23C on the basis of a request signal from the facility device 30.

In the present embodiment, the value exchange program 23B illustrated in Figure 3 provides content provision compensation information c1, which is compensation for provision of stimulus information, via the terminal 70, to the content provider 5 that provides a new content to be added and stored in the first storage area 23C.

In the present embodiment, the content provision compensation information c1 is money or information regarding a status of provision of the new content to the care-needing person 4 via the facility device 30. The money includes legal tender of each country, virtual currency, and the like, but is not limited thereto, and also includes, for example, electronic money allowing electronic settlement.

When the content provision compensation information c1 is the money, it is assumed that the content provision compensation information c1 is provided, to the terminal 70, as remittance information indicating that the money is remitted to an account of the financial institution of the content provider 5.

In the present embodiment, the content provision compensation information c1 to be provided to the content provider 5 is determined on the basis of a specific response of the care-needing person 4 that is detected by the response detection unit (which will be described later) of the facility device 30 or the number of times that a new content is provided to the care-needing person 4 via a content provision unit (which will be described later) of the facility device 30.

For example, when the specific resonance of the care-needing person 4 that is detected by the resonance detection unit is great or when the number of times that a new content is provided to the care-needing person 4 is large, the amount of money to be provided as the content provision compensation information c1 increases.

On the other hand, for example, when the content provision compensation information c1 is determined on the basis of the number of times that a new content is provided to the care-needing person 4, the information regarding a status of provision of the new content may be provided as the content provision compensation information c1.

Furthermore, in the present embodiment, the value exchange program 23B provides, to the care facility 2, use compensation information c2 that requests use compensation via the facility device 30 on the basis of access of the care assistant 3 of the care facility 2 to the first storage area 23C of the server 20 via the facility device 30.

In the present embodiment, the use compensation information c2 is money, and in this case, it is assumed that the use compensation information c2 is provided, to the facility device 30, as remittance information indicating that the money is remitted to an account of the financial institution of the operator 1.

Here, for example, it is assumed that after the care facility 2 remits the money to the operator 1 on the basis of the use compensation information c2, a part or all of the expense requested as the use compensation is refunded by insurance to be paid to the care-needing person 4 from a business entity carrying on life insurance business, long-term care insurance business, or non-life insurance business or the social insurance medical fee payment funds, and a grant-in-aid from the national or local government or the like to the care-needing person 4.

Figure 5 is a block diagram illustrating a schematic configuration of the first storage area 23C. As illustrated in the figure, the first storage area 23C includes a first content library 23Ca, a second content library 23Cb, and a third content library 23Cc.

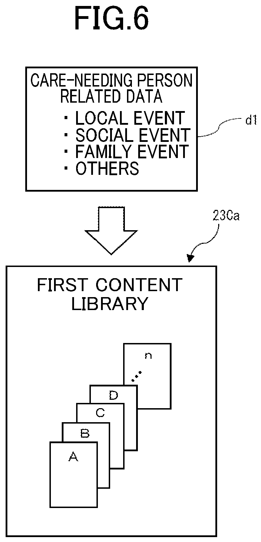

Figure 6 is a block diagram illustrating a schematic configuration of the first content library 23Ca. As illustrated in the figure, the first content library 23Ca is constituted by a plurality of first contents A to n which are pieces of first stimulus information.

In the present embodiment, from among the first contents A to n, contents enabling mental tension of the care-needing person 4 to be released are selected on the basis of care-needing person related data d1 such as "local events" of a hometown of the care-needing person 4, "social events" in which the society and the social condition during the childhood of the care-needing person 4 are reflected, or "family events" regarding a family of the care-needing person 4.

Specifically, the "local events" are, for example, assumed to be images of school sports and school events when the care-needing person 4 was an elementary school student, the "social events" are, for example, assumed to be images of the Tokyo Olympic Games 1964 that was held during the childhood of the care-needing person 4, and the "family events" are, for example, assumed to be images taken when the care-needing person 4 went on a trip with his/her family, and daily images taken with his/her family.

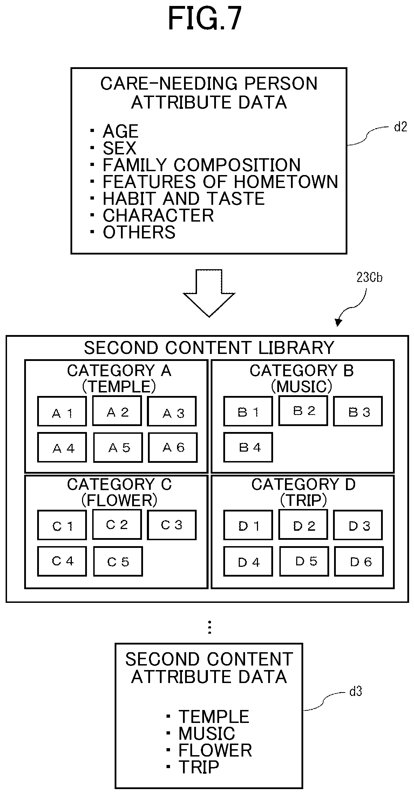

Figure 7 is a block diagram illustrating a schematic configuration of the second content library 23Cb. As illustrated in the figure, the second content library 23Cb is constituted by a plurality of second contents A1 to D6 which are pieces of second stimulus information.

In the present embodiment, from among the second contents A1 to D6, contents enabling an interest of the care-needing person 4 to be aroused are selected on the basis of care-needing person attribute data d2 constituted by attributes of the care-needing person 4 such as "age," "sex," "family composition," "features of hometown," or "habit and taste" of the care-needing person 4.

The care-needing person attribute data d2 is generated by hearings with the family of the care-needing person 4, for example.

In the present embodiment, the second contents A1 to A6 of the second contents A1 to D6 include images or a video of a certain temple where the care-needing person 4 has visited, which are selected on the basis of the care-needing person attribute data d2, and, for example, the second content A1 includes images of Kiyomizu-dera temple (Kyoto), and the second content A2 includes images of Horyu-ji temple (Nara).

The second contents A1 to A6 are classified as a "category A" based on the attribute "temple" of the second contents A1 to A6 in a category hierarchy, and are stored, as the second content library 23Cb, in the first storage area 23C.

In the present embodiment, the second contents B1 to B4 of the second contents A1 to D6 include videos relating favorite music of the care-needing person 4, which are selected on the basis of the care-needing person attribute data d2, and, for example, the second content B1 includes a video in which a musical piece 1 is being played by Taishogoto, and the second content B2 includes a video in which a musical piece 2 is being played by Taishogoto.

The second contents B1 to B4 are classified as a "category B" based on the attribute "music" of the second contents B1 to B4 in the category hierarchy, and are stored, as the second content library 23Cb, in the first storage area 23C.

In the present embodiment, the second contents C 1 to C5 of the second contents A1 to D6 include images or videos of favorite flower of the care-needing person 4 and an aroma having the smell of the flower, which are selected on the basis of the care-needing person attribute data d2, and, for example, the second content C1 includes an image of rose and an aroma having the smell of rose, and the second content C2 includes an image of cosmos and an aroma having the smell of cosmos.

The second contents C 1 to C5 are classified as a "category C" based on the attribute "flower" of the second contents C 1 to C5 in a category hierarchy, and are stored, as the second content library 23Cb, in the first storage area 23C.

Furthermore, in the present embodiment, the second contents D 1 to D6 of the second contents A1 to D6 include videos and images taken when the care-needing person 4 went on a trip, which are selected on the basis of the care-needing person attribute data d2, and, for example, the second content D1 includes a video taken when the care-needing person 4 went on a trip to Kumamoto, and the second content D2 includes a video taken when the care-needing person 4 went on a trip to Hong Kong.

The second contents D 1 to D6 are classified as a "category D" based on the attribute "trip" of the second contents D 1 to D6 in the category hierarchy, and are stored, as the second content library 23Cb, in the first storage area 23C.

In the present embodiment, the attributes "temple," "music," "flower," and "trip" of the second contents A1 to D6 are recognized as the second content attribute data d3, and the second content attribute data d3 is assigned to the second contents A1 to D6 as metadata.

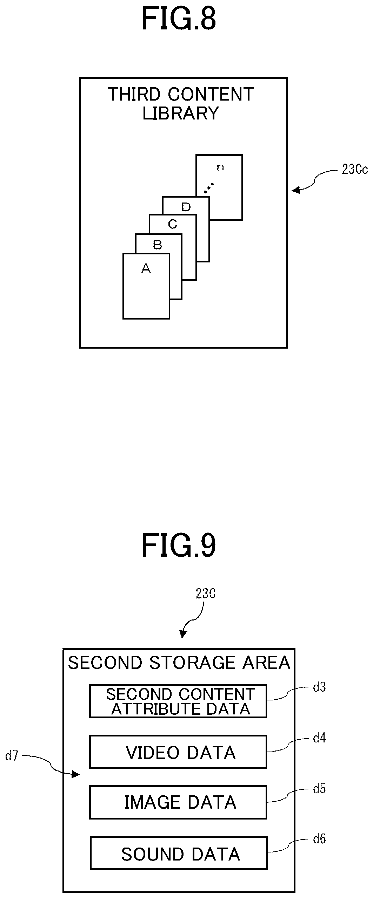

Figure 8 is a block diagram illustrating a schematic configuration of the third content library 23Cc. As illustrated in the figure, the third content library 23Cc is constituted by a plurality of third contents A to n which are pieces of third stimulus information.

In the present embodiment, from among the third contents A to n, contents enabling a care-needing person 4 to be cooled down is selected, the care-needing person 4 being engaged in the second contents A1 to D6.

Here, the term "cool" means causing a care-needing person 4 to be physically and mentally cooled down, the care-needing person 4 showing a specific response such as uttering words, and clapping of hands by arousing an interest in the second contents A1 to D6, and the third contents A to n enabling the care-needing person 4 to be cooled down are, for example, assumed to be images of a dog or a cat, images of natural scenery, scenery of seasons (cherry blossom, snow, flower, and the like), and images of any place such as a famous place and a historic spot.

Figure 9 is a block diagram illustrating a schematic configuration of the second storage area 23D. As illustrated in the figure, the second storage area 23D stores, as care-needing person data d7, the second content attribute data d3, and video data d4, image data d5 and sound data d6 of the care-needing person 4 that are detected by the response detection unit (which will be described later) of the facility device 30.

The care-needing person data d7 can be used as the learning data for machine learning, for example.

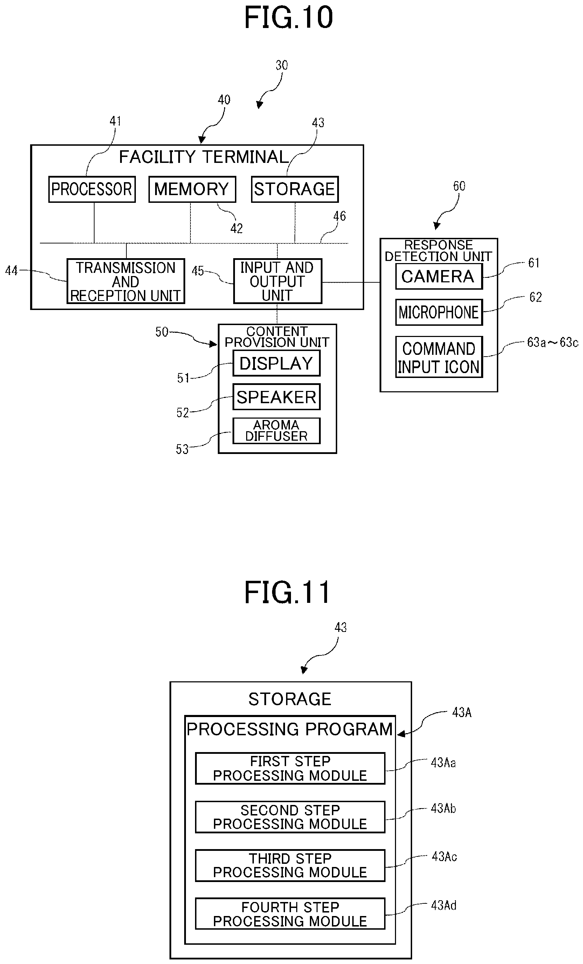

Figure 10 is a diagram illustrating a schematic configuration of the facility device 30 of the care-needing person assistance system 10 according to the present embodiment. As illustrated in the figure, the facility device 30 includes a facility terminal 40, a content provision unit 50 which is the stimulus information provision means, and a response detection unit 60 which is the response detection means.

In the present embodiment, the facility device 30 is implemented by an information processing device such as a so-called desktop or notebook computer, or a so-called tablet-type personal digital assistant.

The facility terminal 40 includes, as main components, a processor 41, a memory 42, a storage 43, a transmission and reception unit 44, and an input and output unit 45, and these components are electrically connected to one another via a bus 46.

The processor 41 is a computing device that controls the operation of the facility terminal 40 to control exchange of data between elements and perform processing required to execute an application program, for example.

In the present embodiment, the processor 41 is, for example, a central processing unit (CPU), and executes an application program and the like that are stored in the storage 43 and loaded into the memory 42 to perform the processing (which will be described later).

The memory 42 includes a main storage device constituted by a volatile storage device such as a dynamic random access memory (DRAM), and an auxiliary storage device constituted by a nonvolatile storage device such as a flash memory or a hard disk drive (HDD).

The memory 42 is used as a work area of the processor 41, and stores a basic input/output system (BIOS) to be executed when the facility terminal 40 starts up, various pieces of setting information, and the like.

The storage 43 stores an application program, data used for various types of processing, and the like. In the present embodiment, the storage 43 stores feature amount data including the amount of sound uttered by the care-needing person 4, and the behavior of the care-needing person 4, and stores a processing program for performing various types of processing. The details of the processing program will be described later.

The transmission and reception unit 44 connects the server 20 to the Internet 100. The transmission and reception unit 44 may include a short-range communication interface such as Bluetooth (registered trademark) and Bluetooth Low Energy (BLE).

In the present embodiment, the facility terminal 40 is connected to the server 20 via the transmission and reception unit 44 and the Internet 100.

The input and output unit 45 is connected with an information input device such as a keyboard and a mouse, and in the present embodiment, the input and output unit 45 is further connected with the content provision unit 50 and the response detection unit 60.

The bus 46 communicates, for example, an address signal, a data signal, and various control signals among the processor 41, the memory 42, the storage 43, the transmission and reception unit 44, and the input and output unit 45 that are connected to the bus 26.

The content provision unit 50 includes a display 51, a speaker 52, and an aroma diffuser 53. The display 51 displays a content including a video or an image from among the first contents A to n, the second contents A1 to D6, and the third contents A to n.

The speaker 52 produces a content composed of music or sound, or music or sound of the content including the music or sound from among the first contents A to n, the second contents A1 to D6, and the third contents A to n.

In the present embodiment, the aroma diffuser 53 stores an aroma, and produces a content composed of smell or smell of a content emitting the smell from among the first contents A to n, the second contents A1 to D6, and the third contents A to n.

In the present embodiment, the response detection unit 60 includes a camera 61 which is an image capture device, a microphone 62 which is a sound pickup device, and command input icons 63a to 63c to be displayed on the display 51.

The camera 61 captures the care-needing person 4 as a video or an image, and the microphone 62 picks up a voice emitted by the care-needing person 4 or the other sounds.

In the present embodiment, the camera 61 and the microphone 62 detect the responses of the care-needing person 4 when the care-needing person 4 shows specific responses such as uttering words and gazing at the display 51 at the time when any content is provided to the care-needing person 4, and a second step processing module (which will be described later) transmits a detection signal to the server 20 when the camera 61 and the microphone 62 detect the responses.

In the present embodiment, the specific responses of the care-needing person 4 that are detected by the camera 61 and the microphone 62 are stored, as the video data d4, the image data d5, and the sound data d6, in the second storage area 23D.

The details of the command input icons 63a to 63c will be described later.

Figure 11 is a diagram illustrating a schematic configuration of the storage 43 of the facility terminal 40. As illustrated in the figure, a processing program 43A is stored in the storage 43, and the processing program 43A includes a first step processing module 43Aa, a second step processing module 43Ab, a third step processing module 43Ac, and a fourth step processing module 43Ad.

In the present embodiment, the first step processing module 43Aa is a module that transmits, to the server 20, a request signal that requests to extract any first contents A to n from the first content library 23Ca stored in the first storage area 23C, and displays the extracted first contents A to n on the display 51.

In the present embodiment, the second step processing module 43Ab is a module that transmits, to the server 20, a request signal that requests to extract any second contents A1 to D6 from the second content library 23Cb stored in the first storage area 23C, and displays the extracted second contents A1 to D6 on the display 51.

In the present embodiment, the second step processing module 43Ab determines whether the response of the care-needing person 4 detected by the camera 61 and the microphone 62 is a specific response, on the basis of feature amount data including the amount of sound uttered by the care-needing person 4 and the behavior of the care-needing person 4, the feature amount data being stored in the storage 43, and transmits, to the server 20, a detection signal when the second step processing module 43Ab determines that the detected response is the specific response.

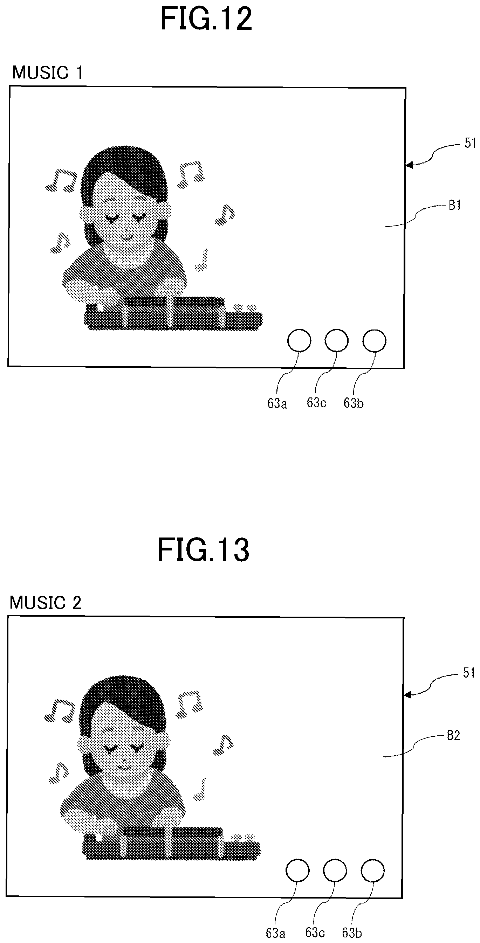

Figure 12 is a diagram illustrating an overview in the case where the second contents A1 to D6 are displayed on the display 51 by the second step processing module 43Ab. As illustrated in the figure, for example, the second content B1 including a video and music in which the musical piece 1 is being played by Taishogoto is displayed on the display 51.

At this time, the command input icons 63a to 63c are displayed on the display 51, and the command input icon 63a indicates "Interested," the command input icon 63b indicates "Non interested," and the command input icon 63c indicates "Neither interested nor not interested."

When the command input icons 63a to 63c are input, the second step processing module 43Ab transmits a detection signal to the server 20 on the basis of the inputs.

As illustrated in Figure 11, in the present embodiment, the third step processing module 43Ac is a module that, when the camera 61 and the microphone 62 detect the specific response of the care-needing person 4, or when the command input icon 63a ("Interested") is input and the other second contents B2 to B4 having the same attribute as the attribute of the second content B1 are extracted from the second content library 23Cb stored in the first storage area 23C, displays the extracted contents on the display 51.

Figure 13 is a diagram illustrating an overview in the case where the second contents A1 to D6 are displayed on the display 51 by the third step processing module 43Ac. As illustrated in the figure, for example, the second content B2 having the same attribute as the attribute of the second content B1, the second content B2 including a video and music in which the musical piece 2 is being played by Taishogoto, is displayed on the display 51.

At this time, the command input icons 63a to 63c are displayed on the display 51.

Furthermore, as illustrated in Figure 11, in the present embodiment, the fourth step processing module 43Ad is a module that transmits, to the server 20, a request signal that requests to extract any third contents A to n from the third content library 23Cc stored in the first storage area 23C, and displays the extracted third contents A to n on the display 51.

In the present embodiment, the terminal 70 illustrated in Figure 1 is implemented by a desktop or notebook computer, but may be implemented by a smartphone which is a personal digital assistant or a tablet computer.

The terminal 70 includes, for example, a processor, a memory, a storage, and a transmission and reception unit, and in the present embodiment, the terminal 70 stores a new content created by the content provider 5 and transmits the stored content to the server 20 of the operator 1.

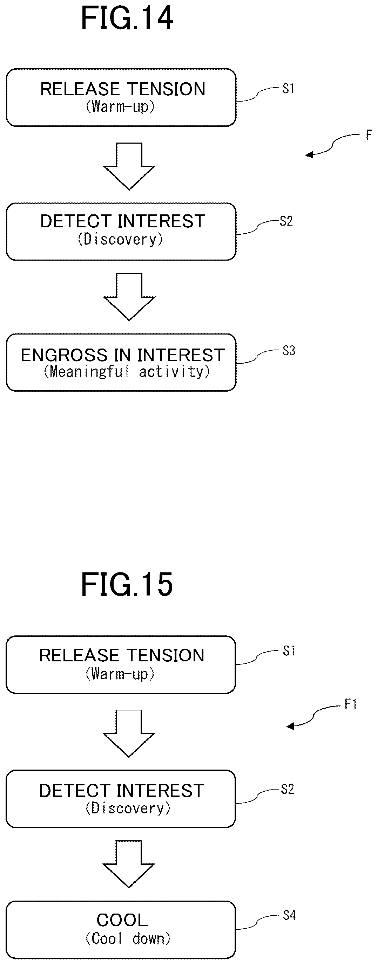

Figure 14 is a diagram illustrating an overview of a care assistance flow to be executed for the purpose of improving behavioral and psychological symptoms of the care-needing person 4. As illustrated in the figure, the care assistance flow F includes a first step S1 of releasing tension of the care-needing person 4 (Warm-up), a second step S2 of detecting an interest of the care-needing person 4 (Discovery), and a third step S3 of causing the care-needing person 4 to be engaged in the interest (Meaningful Activity), and is executed by the care-needing person assistance system 10 of the present embodiment.

First, in the first step S1, the care assistant 3 operates the facility terminal 40 to transmit a request signal that requests the first step execution module 23Aa of the assistance program 23A of the server 20 to extract any first contents A to n from the first content library 23Ca via the first step processing module 43Aa of the processing program 43A.

The first step execution module 23Aa extracts any first contents A to n from the first content library 23Ca on the basis of the request signal. When any of the any first contents A to n is extracted from the first content library 23Ca, the extracted first content A, for example, is displayed on the display 51 by the first step processing module 43Aa to be provided to the care-needing person 4.

In the present embodiment, the first contents A to n are displayed on the display 51 sequentially or in any order at any intervals of time (e.g., 10 seconds).

In the present embodiment, the first contents A to n include, for example, images of school sports when the care-needing person 4 was an elementary school student, and images of the Tokyo Olympic Games that was held during the childhood of the care-needing person 4, whereby the mental tension of the care-needing person 4 can be released (Warm-up).

After the mental tension of the care-needing person 4 is released in the first step S1, in the second step S2, the care assistant 3 operates the facility terminal 40 to transmit a request signal that requests the second step execution module 23Ab of the assistance program 23A of the server 20 to extract any second contents A1 to D6 from the second content library 23Cb via the second step processing module 43Ab of the processing program 43A.

The second step execution module 23Ab extracts any second contents A1 to D6 from the second content library 23Cb on the basis of the request signal. When any of the any second contents A1 to D6 is extracted from the second content library 23Cb, the extracted second content B1, for example, is displayed on the display 51 by the second step processing module 43Ab to be provided to the care-needing person 4.

In the present embodiment, the second contents A1 to D6 are configured to be displayed on the display 51 sequentially or in any order at any intervals of time.

At this time, the second contents A1 to D6 are hierarchized and classified on the basis of attributes of the second contents A1 to D6 and are stored, as the second content library 23Cb, in the first storage area 23C, and therefore a process of displaying the second contents A1 to D6 on the display 51 can be smoothly performed.

In the present embodiment, the second content B1 displayed on the display 51 includes a video and music in which the musical piece 1 is being played by Taishogoto as illustrated in Figure 12, and when the care-needing person 4 who watches the second content B1 shows specific responses such as uttering words and gazing at the display 51, the care-needing person 4 is considered to be interested in "music" as the attribute of the second content B1.

When the camera 61 and the microphone 62 detect the responses of the care-needing person 4, a detection signal is transmitted to the server 20 by the second step processing module 43Ab.

In the present embodiment, the specific responses of the care-needing person 4 that are detected by the camera 61 and the microphone 62 are stored, as the video data d4, the image data d5, and the sound data d6, in the second storage area 23D.

On the other hand, in the present embodiment, in the care where the care-needing person 4 who watches the second content B1 shows the above-described specific responses, the care assistant 3 can input the command input icon 63a ("Interested").

When the care assistant 3 inputs the command input icon 63a, a detection signal is transmitted to the server 20 by the second step processing module 43Ab.

When the detection signal is thus transmitted to the server 20 by the second step processing module 43Ab, the "music" as the attribute of the second content B1 to which the care-needing person 4 shows the specific responses is detected, as the second content attribute data d3, by the second step execution module 23Ab, and is stored in the second storage area 23D. In this way, the interest of the care-needing person 4 is detected (Discovery).

In the present embodiment, in either of the case where the detection signal is transmitted on the basis of the detection by the camera 61 and the microphone 62 or the case where the detection signal is transmitted in response to input of the command input icon 63a, the second content attribute data d3 is detected by the second step execution module 23Ab.

In the case where the care-needing person data d7 is used as the learning data for machine learning, it can be configured that the second contents A1 to D6 having the attribute in which the care-needing person 4 shows an interest are optimized by, for example, an artificial intelligence program to be displayed on the display 51.

When the second content attribute data d3 is detected, in the third step S3, the other second contents B2 to B4 having the same attribute as the attribute of the second content B1 are extracted from the second content library 23Cb stored in the first storage area 23C by the third step execution module 23Ac.

At this time, the other second contents B2 to B4 having the same attribute as the attribute of the second content B1, the other second contents B2 to B4 being extracted from the second content library, are displayed on the display 51 by the third step processing module 43Ac to be provided to the care-needing person 4.

In the present embodiment, the second content B2 of the second contents B2 to B4 displayed on the display 51 includes a video and music in which the musical piece 2 is being played by Taishogoto as illustrated in Figure 13, and the second contents B3, B4 also include videos and music in which a musical piece 3, a musical piece 4 are being played by Taishogoto, respectively, for example.

If a care-needing person 4 who is considered to be interested in "music" as the attribute of the second content B1 imitates the motion of playing Taishogoto or has behavior of clapping hands to the music, for example, when the other second contents B2 to B4 having the same attribute as the attribute of the second content B1 are provided to the care-needing person 4, it can be said that the care-needing person 4 is in a state of being engaged in the music (Meaningful Activity).

In the case where the care-needing person 4 who watches the second content B1 does not show the above-described specific responses within any time period, the care-needing person 4 is considered not to be interested in "music" as the attribute of the second content B1, and therefore the camera 61 and the microphone 62 do not detect the specific responses of the care-needing person 4.

In this case, in the present embodiment, in the second step S2, until any second contents A1 to D6 are extracted from the second content library 23Cb and the care-needing person 4 shows the above-described specific responses, the extracted any second contents A1 to D6 are automatically displayed on the display 51 sequentially or in any order at any intervals of time to be provided to the care-needing person 4.

On the other hand, in the present embodiment, in the care where the care-needing person 4 who watches the second content B 1 does not show the above-described specific responses, the care assistant 3 can input the command input icon 63b ("Non interested") or the command input icon 63c ("Neither interested nor not interested").

In this case, until the care-needing person 4 shows the above-described specific responses, in the second step S2, any second contents A1 to D6 are extracted from the second content library 23Cb by operation of the care assistant 3 on the facility terminal 40, the extracted any second contents A1 to D6 are displayed on the display 51 to be provided to the care-needing person 4.

In executing such care assistance flow F, a care assistance preparation flow F1 illustrated in Figure 15 is executed after a care-needing person 4 moves into the care facility 2 from the standpoint of smoothly and rapidly executing the care assistance flow F.

As illustrated in the figure, the care assistance preparation flow F1 includes a first step S1 of releasing tension of the care-needing person 4 (Warm-up), a second step S2 of detecting an interest of the care-needing person 4 (Discovery), and a fourth step S4 of causing the care-needing person 4 to be cooled down (Cool down).

After the mental tension of the care-needing person 4 is released in the first step S1, and the second content attribute data d3 of any of the second contents A1 to D6 to which the care-needing person 4 shows the specific response is detected and the interest of the care-needing person 4 is detected in the second step S2, the fourth step S4 is executed.

In the fourth step S4, the care assistant 3 operates the facility terminal 40 to transmit a request signal that requests the fourth step execution module 23Ad of the assistance program 23A of the server 20 to extract any third contents A to n from the third content library 23Cc via the fourth step processing module 43Ad of the processing program 43A.

The third step execution module 23Ac extracts any third contents A to n from the third content library 23Cc on the basis of the request signal. When any of the any third contents A to n is extracted from the third content library 23Cc, the third content A, for example extracted is displayed on the display 51 by the third step processing module 43Ac to be provided to the care-needing person 4.

In the present embodiment, the third contents A to n are configured to be displayed on the display 51 sequentially or in any order at any intervals of time (e.g., 10 seconds).

In the present embodiment, the third contents A to n include images of a dog or a cat, images of natural scenery, scenery of seasons (cherry blossom, snow, and the like), and images of a certain place, and the third contents A to n are used to cause the care-needing person 4 to be cooled down (Cool down), the care-needing person 4 being engaged in the second contents A1 to D6, and then the care assistance preparation flow F1 ends.

The care assistance preparation flow F1 is executed in a relatively short time (e.g., about 30 minutes) as part of an orientation performed when the care-needing person 4 moves into the care facility 2, for example, and may be executed a plurality of times over a period of several days until the interest of the care-needing person 4 is detected.

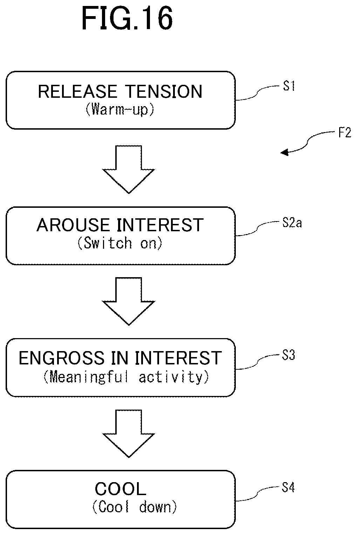

On the other hand, when the interest of the care-needing person 4 is detected in the care assistance preparation flow F1, a periodic care assistance flow F2 illustrated in Figure 16 is routinely executed as a part of the care assistance flow F.

As illustrated in the figure, the care assistance preparation flow F2 includes a first step S1 of releasing tension of the care-needing person 4 (Warm-up), an interest arousing step S2a of arousing an interest of the care-needing person 4 (Switch on), a third step S3 of causing the care-needing person 4 to be engaged in the interest (Meaningful Activity), and a fourth step S4 of causing the care-needing person 4 to be cooled down (Cool down).

After releasing the mental tension of the care-needing person 4 in the first step S1, the interest arousing step S2a is executed. In the interest arousing step S2a, for example, the second content B1 to which the care-needing person 4 shows the specific responses is displayed on the display 51 to cause the care-needing person 4 to show the response in the second step S2, and the interest of the care-needing person 4 in the attribute ("music") recognized as the second content attribute data d3 is aroused (Switch on).

The execution procedure of the interest arousing step S2a is the same as the procedure executed in the second step S2.

After the interest of the care-needing person 4 is aroused in the interest arousing step S2a, the third step S3 is executed to cause the care-needing person 4 to be engaged in, for example, the second content B2 regarding the music, thereafter, the fourth step S4 is executed to cause the care-needing person 4 to be cooled down, and the periodic care assistance flow F2 ends.

In this way, according to the present embodiment, the content provider 5 can provide a new content to be provided to the care-needing person 4, and therefore, the contents can be collected in a wide range without relying on only the assistant and family of the care-needing person 4 in collecting the contents.

In this case, the content provision compensation information C1 such as money is provided to the content provider 5, which makes it possible to provide an incentive for providing a new content to the content provider 5.

Accordingly, the contents can be efficiently collected.

On the other hand, in assisting the care-needing person 4, providing, to the care-needing person 4, the contents that stimulate at least one of five senses makes it possible to properly detect the care-needing person 4 showing the specific responses to which attribute of the second contents A1 to D6 by what sense in the second step S2.

Furthermore, in the third step S3, providing the other second content B2, for example, having the same attribute as the attribute of the second content B 1, for example, to which the care-needing person 4 shows the specific responses makes it possible to cause the care-needing person 4 to be engaged in the second content B2.

Accordingly, causing the care-needing person 4 to be engaged in the second content B2 having the interesting attribute makes it possible to promote a spontaneous action regarding the attribute (e.g., "music"), and therefore, the behavioral and psychological symptoms of the care-needing person can be properly improved by attempting the brain function activation through the five senses of the care-needing person 4 without resorting to drug therapy.

The care-needing person assistance system 10 can interrupt or discontinue the use on the basis of the determination of the family of the care-needing person 4 or the care assistant 3 who observed the response of the care-needing person 4, and therefore the possibility that the use of the care-needing person assistance system 10 adversely affects the care-needing person 4 is extremely low, whereby the care-needing person assistance system 10 can be used without anxiety according to the condition of the care-needing person 4.

On the other hand, even when the drug therapy is applied to the care-needing person 4, the behavioral and psychological symptoms of the care-needing person 4 are expected to be improved by using the care-needing person assistance system 10 together with the drug therapy.

Furthermore, the family of the care-needing person 4 watches the contents to be provided to the care-needing person 4 by the care-needing person assistance system 10 to share the contents with the family, whereby good communication between the care-needing person 4 and the family thereof can be promoted, the isolation of the care-needing person 4 from the family can be eliminated, and mutual understanding between the care-needing person 4 and the family can be improved, which is advantageous to both of the care-needing person 4 and the family thereof.

That is, the care-needing person assistance system 10 of the present embodiment can be recognized as a communication means or an information sharing means that promotes the communication or mutual understanding between the care-needing person 4 and the family thereof or the care assistant 3.

In addition, the second step S2 is executed in the state in which the mental tension of the care-needing person 4 is released by the first step S1, which makes it possible to accurately detect the attribute in the second contents A1 to D6 to which the care-needing person 4 shows the specific response.

Furthermore, the second contents A1 to D6 are hierarchized on the basis of attributes of the second contents A1 to D6 and are stored in the first storage area 23C, and therefore a process of displaying the second contents A1 to D6 on the display 51 can be smoothly performed.

On the other hand, when, in the care facility 2, the care assistant 3 accesses the first storage area 23C of the server 20 of the operator 1 via the facility device 30, the use compensation information c2 that requests use compensation from the care facility 2 is provided, whereby it is expected to contribute to business activities of the operator 1.

As described above, the care-needing person assistance system 10 according to the present embodiment is defined as an assistance method of a care-needing person using the care-needing person assistance system 10, non-drug therapy using the care-needing person assistance system 10, a therapeutic method using the care-needing person assistance system 10, or the use of the care-needing person assistance system 10, and therefore the present embodiment and all of the effects thereof are comprehended.

Note that the present invention is not limited to the above-described embodiment, and can be variously modified without departing from the scope of the present invention.

In the above-described embodiment, there has been described the case where the use compensation information c2 is provided to the care facility 2 when the care assistant 3 accesses the first storage area 23C of the server 20 of the operator 1 via the facility device 30, but it may be configured to provide the use compensation information c2 to the care facility 2 not on the basis of the access to the first storage area 23C but on the basis of the usage frequencies of the content provision unit 50 and the response detection unit 60 of the facility device 30.

In the above-described embodiment, there has been described the case where the response detection unit 60 includes the camera 61, the microphone 62, and the command input icons 63a to 63c, but the response detection unit 60 may be configured to include any one of a set of the camera 61 and the microphone 62, and a set of the command input icons 63a to 63c.

Furthermore, the response detection unit 60 may be configured to include an acceleration sensor, a heart rate detection sensor, or the like to detect the amount of movement of the care-needing person 4.

In the above-described embodiment, there has been described the case where the first contents A to n, the second contents A1 to D6, and the third contents A to n include contents that stimulate a visual sense, an auditory sense, or an olfactory sense among the five senses of the care-needing person 4, but may have contents that stimulate a tactile sense, and a gustatory sense.

In the above-described embodiment, there has been described the case where the contents are provided to the care-needing person 4 by accessing the server 20 via the facility device 30, but it may be configured to provide the contents to the care-needing person 4 after the contents or an assistance program for providing the contents are downloaded on the facility device 30.

In the above-described embodiment, there has been described the case where the care assistant 3 operates the facility device 30 to cause the contents to be displayed on the display 51 of the facility device 30 and to be provided to the care-needing person 4, but it may be configured to separately provide a device operated by the care assistant 3 and a device for providing the contents to the care-needing person 4 so that these devices constitute the facility device 30.

In this case, the care assistant 3 and the care-needing person 4 may use the device while having face-to-face contact with each other in the same space, to use the care-needing person assistance system 10, or the device for the care assistant 3 and the device for the care-needing person 4 may be connected to each other by a known online tool to use the care-needing person assistance system 10 in the remote environment.

For example, even when the care-needing person 4 cannot make face-to-face contact with the family thereof or the care assistant 3 due to behavioral restrictions on infection spread or the like, the family of the care-needing person 4 or the care assistant 3 can contact the care-needing person 4 via the care-needing person assistance system 10.

Accordingly, a feeling of anxiety of the care-needing person 4 and the family of the care-needing person 4 or the care assistant 3 can be eliminated, the feeling of anxiety being assumed to be caused when the care-needing person 4 and the family thereof or the care assistant 3 cannot communicate with each other.

Note that the device operated by the care assistant 3 and the device for providing the contents to the care-needing person 4 may be implemented by an information processing device such as a so-called desktop or notebook computer, or a so-called tablet-type personal digital assistant in the same manner as in the above-described embodiment.

In the above-described embodiment, there has been described the case where the user is the care facility 2, but may be an individual that can care for the care-needing person 4. In this case, the contents may be provided to a residence of the care-needing person 4.

### (Reference Example 1)

In the above-described embodiment, there has been described the case where the care assistance flow F and the periodic care assistance flow F2 are executed using the care-needing person assistance system 10, but the care assistant 3 may create a care plan on the basis of the attribute detected in the second step S2 so that the third step S3 can be routinely executed on the basis of the care plan.

### (Reference Example 2)

In the above-described embodiment, there has been described the case where the care-needing persons 4 having the behavioral and psychological symptoms are targeted, but it is expected that, also with respect to care-needing persons having autism, autism can be improved by executing the first step to the third step using the care-needing person assistance system having the same configuration as the care-needing person assistance system 10 of the above-described embodiment in a special nursing care facility or the like.

### Reference Signs List

- 1: Operator
- 2: Care facility (user)
- 3: Care assistant
- 4: Care-needing person
- 5: Content provider (provider)
- 10: Care-needing person assistance system
- 20: Server
- 23A: Assistance program
- 23B: Value exchange program (value exchange means)
- 23C: First storage area (stimulus information storage means)
- 30: Facility device
- 43A: Processing program
- 50: Content provision unit (stimulus information provision means)
- 60: Response detection unit (response detection means)
- 61: Camera (image capture device)
- 62: Microphone (sound pickup device)
- 70: Terminal
- A to n: First content (first stimulus information), Third content (third stimulus information)
- A1 to D6: Second content (second stimulus information)
- c1: Content provision compensation information (stimulus information provision compensation)
- c2: Use compensation information (use compensation)
- d3: Second content attribute data

## Claims

1. A care-needing person assistance system comprising:
a stimulus information storage means that stores stimulus information about a stimulus for at least one of five senses of a care-needing person; and
a value exchange means that provides compensation for provision of stimulus information to a provider who provides new stimulus information to be additionally stored in the stimulus information storage means.

2. The care-needing person assistance system according to claim 1, wherein the stimulus information is at least one of a video, an image, music, a sound and an aroma.

3. The care-needing person assistance system according to claim 1 or 2, further comprising:
a stimulus information provision means that provides, to the care-needing person, the stimulus information stored in the stimulus information storage means; and
a response detection means that detects a response of the care-needing person to the stimulus by the stimulus information provided by the stimulus information provision means.

4. The care-needing person assistance system according to claim 3, wherein the compensation for provision of stimulus information to be provided to the provider is determined on a basis of a specific response of the care-needing person detected by the response detection means.

5. The care-needing person assistance system according to claim 3, wherein the compensation for provision of stimulus information to be provided to the provider is determined on a basis of a number of times that the new stimulus information is provided to the care-needing person via the stimulus information provision means.

6. The care-needing person assistance system according to any one of claims 3 to 5, wherein the compensation for provision of stimulus information is money and/or information regarding a status of provision of the new stimulus information to the care-needing person via the stimulus information provision means.

7. The care-needing person assistance system according to any one of claims 3 to 5, wherein the value exchange means requests use compensation from a user on a basis of access of the user to the stimulus information storage means.

8. The care-needing person assistance system according to claim 7, wherein the use compensation is money.

9. The care-needing person assistance system according to claim 3, wherein the care-needing person assistance system executes:
a first step of providing, from among pieces of the stimulus information stored in the stimulus information storage means, first stimulus information enabling mental tension of the care-needing person to be released;
a second step of providing, from among the pieces of the stimulus information, any pieces of second stimulus information of pieces of second stimulus information stored in the stimulus information storage means on a basis of an attribute of the care-needing person and detecting an attribute of a piece of the second stimulus information to which the care-needing person responds, from among the any pieces of the second stimulus information provided; and
a third step of providing, to the care-needing person, another piece of the second stimulus information having the same attribute as the attribute of the piece of the second stimulus information detected in the second step.

10. The care-needing person assistance system according to claim 9, wherein the care-needing person assistance system executes a periodic care assistance flow in which, after providing the piece of the second stimulus information detected in the second step to cause the care-needing person to respond to the piece of the second stimulus information and arousing an interest of the care-needing person in the attribute of the piece of the second stimulus information, the other piece of the second stimulus information is provided so that the care-needing person is engaged in the other piece of the second stimulus information.

11. The care-needing person assistance system according to claim 10, wherein in the periodic care assistance flow, the first stimulus information is provided to the care-needing person before the second stimulus information is provided to the care-needing person.

12. The care-needing person assistance system according to claim 10 or 11, wherein in the periodic care assistance flow, third stimulus information enabling the care-needing person to be cooled down is provided after the other piece of the second stimulus information is provided to the care-needing person.
